# EUROPEAN PATENT APPLICATION

(11) **EP 1 920 654 A1**
(43) Date of publication of application: **14.05.2008**
(21) Application number: 06019152.5
(22) Date of filing: 13.09.2006
(51) Int. Cl.: A01N 43/90, C07D 471/04

(54) **Novel pyridopyrazine N-oxides**

(71) Applicant: Syngenta Participations AG, 4058 Basel (CH); Syngenta Limited, Guildford Surrey GU2 7YH (GB)
(72) Inventor: The designation of the inventor has not yet been filed
(74) Representative: Arunasalam, Velautha-Cumaran

(57) **Abstract**

The present invention relates to novel N -oxide derivatives of pyridopyrazines of formula I as active ingredients which have microbiocidal activity, in particular fungicidal activity: 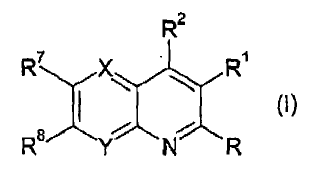 wherein
X is N⁺-O⁻ and Y is N; or
X is N and Y is N⁺-O⁻; or
X and Y are both N⁺-O⁻;
R is for example H, halo, cyano, C₁₋₆ alkyl or C₁₋₆alkoxy;
R¹ is aryl or heteroaryl;
R² is for example halo, C₁₋₈ alkyl, C₃₋₈ cycloalkyl, aryl, or heteroaryl;
R³ and R⁴ independently of each other are for example H, C₁₋₆ alkyl, halo(C₁₋₆)alkyl, C₂₋₈ alkenyl, C₂₋₈ alkynyl or aryl; or
R³ and R⁴ together with the nitrogen atom to which they are attached form a C₃₋₁₀ alkylene chain or C₃₋₇ alkenylene chain optionally substituted with halo, C₁₋₄alkyl, C₁₋₄haloalkyl, C₁₋₄ alkoxy; or
R³ and R⁴ together with the nitrogen atom to which they are attached form for example a morpholine, thiomorpholine or thiomorpholine S-oxide ring; or
R³ and R⁴ together with the nitrogen atom to which they are attached form a bicyclic annelated or fused ring; and
R⁷ and R⁸ independently of each other are for example H, halo, cyano, C₁₋₄alkyl, C₁₋₄alkoxy or halo(C₁₋₄)alkyl.

## Description

The present invention relates to novel N-oxide derivatives of pyridopyrazines as active ingredients which have microbiocidal activity, in particular fungicidal activity. The invention also relates to preparation of these active ingredients, to novel heterocyclic derivatives used as intermediates in the preparation of these active ingredients, to preparation of these novel intermediates, to agrochemical compositions which comprise at least one of the novel active ingredients, to preparation of these compositions and to use of the active ingredients or compositions in agriculture or horticulture for controlling or preventing infestation of plants, harvested food crops, seeds or non-living materials by phytopathogenic microorganisms, preferably fungi.

Derivatives of pyridopyrazines are known in the chemical literature, for example from J. Med. Chem. (1968), 11(6), 1216-18, J. Med. Chem. (1970), 13(5), 853-7 and US 3984412. Examples of recent patent publications include EP-A-1249452, WO02051845, WO02083676, WO02083677, WO02088125, WO04056825, WO05123733 and WO05010000.

The present invention provides a compound of formula I: 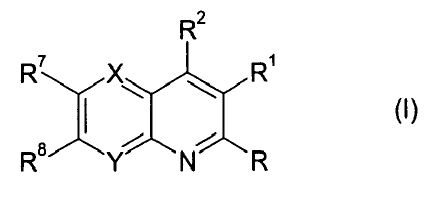 wherein
X is N⁺-O⁻and Y is N; or
X is N and Y is N⁺-O⁻; or
X and Y are both N⁺-O⁻;
R is H, halo, cyano, C₁₋₆ alkyl, C₁₋₆ alkoxy, C₁₋₆ alkylthio, halo(C₁₋₆)alkyl, C₁₋₆ alkenyl,
C₁₋₆ alkynyl or NR³R⁴;
R¹ is aryl or heteroaryl;
R² is halo, C₁₋₈ alkyl, C₃₋₈ cycloalkyl, aryl, heteroaryl, OR³, SR³ or NR³R⁴;
R³ and R⁴ independently of each other are H, C₁₋₈ alkyl, halo(C₁₋₈)alkyl, C₂₋₈ alkenyl, C₂₋₈ alkynyl, aryl, aryl(C₁₋₈)alkyl, C₃₋₈ cycloalkyl, C₃₋₈ cycloalkyl(C₁₋₆)alkyl, heteroaryl, heteroaryl(C₁₋₈)alkyl, NR⁵R⁶, provided that not both R³ and R⁴ are NR⁵R⁶; or
R³ and R⁴ together with the nitrogen atom to which they are attached form a C₃₋₁₀ alkylene chain or C₃₋₇ alkenylene chain optionally substituted with halo, C₁₋₄ alkyl, C₁₋₄ haloalkyl, C₁₋₄ alkoxy; or
R³ and R⁴ together with the nitrogen atom to which they are attached form a morpholine, thiomorpholine, thiomorpholine S-oxide or thiomorpholine S-dioxide ring or a piperazine or piperazine *N*-(C₁₋₄)alkyl (especially *N*-methyl) ring; or
R³ and R⁴ together with the nitrogen atom to which they are attached form a bicyclic annelated or bridged ring;
R⁵ and R⁶ independently of each other are H, C₁₋₈ alkyl, C₂₋₈ alkenyl, C₂₋₈ alkynyl, aryl, aryl(C₁₋₈)alkyl, C₃₋₈cycloalkyl, C₃₋₈cycloalkyl(C₁₋₆)alkyl, heteroaryl or heteroaryl(C₁₋₈)alkyl;
R⁷ and R⁸ independently of each other are H, halo, cyano, C₁₋₄alkyl, C₁₋₄alkoxy, halo(C₁₋₄)alkyl, C₁₋₄alkylthio, C₁₋₄alkylsulphinyl, C₁₋₄alkylsulphonyl, aryl, heteroaryl, halo(C₁₋₆)alkoxy, halo(C₁₋₄)alkylthio, C₂₋₄alkenyl, C₂₋₆alkynyl, C₃₋₆cycloalkyl, C₁₋₆alkoxycarbonyl, R³R⁴NCO or NR⁹R¹⁰;
R⁹ and R¹⁰ independently of each other are H or C₁₋₈ alkyl; or
R⁹ and R¹⁰ together with the nitrogen atom to which they are attached form a C₃₋₁₀ alkylene chain; or
R⁹ and R¹⁰ together with the nitrogen atom to which they are attached form a morpholine, piperazine or piperazine *N*-(C₁₋₄)alkyl ring;
any of the foregoing alkyl, alkenyl, alkynyl or cycloalkyl groups or moieties (other than for R⁷ and R⁸, independently of each other) being optionally substituted with halogen, cyano, C₁₋₆ alkoxy, C₁₋₆ alkylcarbonyl,
C₁₋₆ alkoxycarbonyl, C₁₋₆ haloalkoxy, C₁₋₆ alkylthio, tri(C₁₋₄)alkylsilyl, C₁₋₆ alkylamino or C₁₋₆ dialkylamino;
any of the foregoing morpholine, thiomorpholine, piperidine, piperazine and pyrrolidine rings being optionally substituted with C₁₋₄alkyl (especially methyl); and
any of the foregoing aryl or heteroaryl groups or moieties being optionally substituted with one or more substituents selected from halo, hydroxy, mercapto, C₁₋₆ alkyl, C₂₋₆ alkenyl, C₂₋₆ alkynyl, C₁₋₆ alkoxy, C₂₋₆ alkenyloxy, C₂₋₆ alkynyloxy, halo(C₁₋₆)alkyl, halo(C₁₋₆)alkoxy, C₁₋₆alkylthio, halo(C₁₋₆)alkylthio, hydroxy(C₁₋₆)alkyl, C₁₋₄alkoxy(C₁₋₆)alkyl, C₃₋₆cycloalkyl, C₃₋₆ cycloalkyl(C₁₋₄)alkyl, phenoxy, benzyloxy, benzoyloxy, cyano, isocyano, thiocyanato, isothiocyanato, nitro, -NR"'R"", -NHCOR"', -NHCONR"'R"", -CONR"'R"", -SO₂R"', -OSO₂R"', -COR"', -CR"'=NR"" or -N=CR"'R"", in which R"' and R"" are independently hydrogen, C₁₋₄ alkyl, halo(C₁₋₄)alkyl, C₁₋₄alkoxy, halo(C₁₋₄)alkoxy, C₁₋₄ alkylthio, C₃₋₆ cycloalkyl, C₃₋₆ cycloalkyl(C₁₋₄)alkyl, phenyl or benzyl, the phenyl and benzyl groups being optionally substituted with halogen, C₁₋₄alkyl or C₁₋₄ alkoxy.

Examples of structures embraced by the invention are compounds of formula (II), (III) and (IV) below:

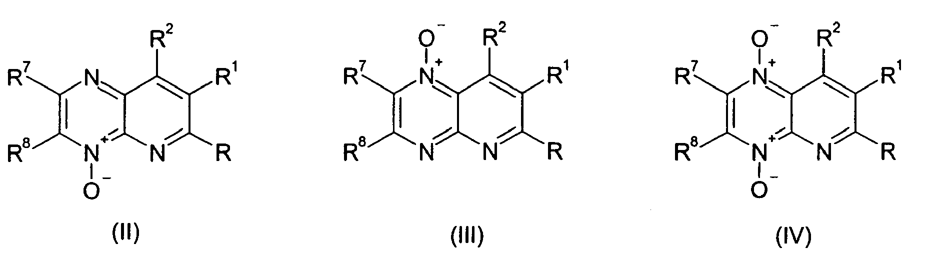

In the above definition aryl includes aromatic hydrocarbon rings like phenyl, naphthyl, anthracenyl, phenanthrenyl and biphenyl, with phenyl being preferred.

Heteroaryl stands for aromatic ring systems comprising mono-, bi- or tricyclic systems wherein at least one oxygen, nitrogen or sulfur atom is present as a ring member. Examples are furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyridazinyl, pyrimidinyl, pyrazinyl, triazinyl, tetrazinyl, indolyl, benzothiophenyl, benzofuranyl, benzimidazolyl, indazolyl, benzotriazolyl, benzothiazolyl, benzoxazolyl, quinolinyl, isoquinolinyl, phthalazinyl, quinoxalinyl, quinazolinyl, cinnolinyl and naphthyridinyl. Each heteroaryl can be linked by a carbon atom or by a nitrogen atom to the pyridopyrazine.

The above aryl and heteroaryl groups may be optionally substituted. This means that they may carry one or more identical or different substituents. Normally not more than three substituents are present at the same time. Examples of substituents of aryl or heteroaryl groups are: halogen, alkyl, haloalkyl, cycloalkyl, cycloalkylalkyl, alkenyl, haloalkenyl, cycloalkenyl, alkynyl, haloalkynyl, alkyloxy, haloalkyloxy, cycloalkoxy, alkenyloxy, haloalkenyloxy, alkynyloxy, haloalkenyloxy, alkylthio, haloalkylthio, cycloalkylthio, alkenylthio, alkynylthio, alkylcarbonyl, haloalkylcarbonyl, cycloalkylcarbonyl, alkenylcarbonyl, alkynylcarbonyl, alkoxyalkyl, cyano, nitro, hydroxy, mercapto, amino, alkylamino, dialkylamino. Typical examples include phenyl, 2-fluorophenyl, 2-chlorophenyl, 2-trifluoromethylphenyl, 2-methylphenyl, 2,3-difluorophenyl, 2,4-difluorophenyl, 2,5-difluorophenyl, 2,6-difluorophenyl, 2,3-dichlorophenyl, 2,4-dichlorophenyl, 2,5-dichlorophenyl, 2,6-dichlorophenyl, 2-chloro-3-fluorophenyl, 2-chloro-4-fluorophenyl, 2-chloro-5-fluorophenyl, 2-chloro-6-fluorophenyl, 3-chloro-2-fluorophenyl, 4-chloro-2-fluorophenyl, 5-chloro-2-fluorophenyl, 2-fluoro-3-trifluoromethylphenyl, 2-fluoro-4-trifluoromethylphenyl, 2-fluoro-5-trifluoromethylphenyl, 2-fluoro-6-trifluoromethylphenyl, 2-chloro-3-trifluoromethylphenyl, 2-chloro-4-trifluoromethylphenyl, 2-chloro-5-trifluoromethylphenyl, 2-chloro-6-trifluoromethylphenyl, 4-fluoro-2-trifluoromethylphenyl, 4-chloro-2-trifluoromethylphenyl, 2-fluoro-3-methylphenyl, 2-fluoro-4-methylphenyl, 2-fluoro-5-methylphenyl, 2-fluoro-6-methylphenyl, 2-chloro-3-methylphenyl, 2-chloro-4-methylphenyl, 2-chloro-5-methylphenyl, 2-chloro-6-methylphenyl, 4-fluoro-2-methylphenyl, 4-chloro-2-methylphenyl, 2,3,4-trifluorophenyl, 2,3,6-trifluorophenyl, 2,4,6-trifluorophenyl, 2;3,4-trichlorophenyl, 2,3,6-trichlorophenyl, 2,4,6-trichlorophenyl, 2,6-difluoro-4-methoxyphenyl, 2,6-difluoro-4-trifluoromethoxyphenyl, 2,6-difluoro-4-trifluoromethylphenyl, 2,6-difluoro-4-cyanophenyl, 2,6-difluoro-4-methylphenyl, 2,6-dichloro-4-methoxyphenyl, 2,6-dichloro-4-trifluoromethoxyphenyl, 2,6-dichloro-4-trifluoromethylphenyl, 2,6-dichloro-4-cyanophenyl, 2,6-dichloro-4-methylphenyl, pentafluorophenyl, 3,5-difluoropyridin-2-yl, 3,5-dichloropyridin-2-yl, 3-chloro-5-fluoropyridine-2-yl, 5-chloro-3-fluoropyridin-2-yl, 3-fluoro-5-trifluoromethylpyridin-2-yl, 3-chloro-5-trifluoromethylpyridin-2-yl, 2,4-difluoropyridin-3-yl, 2,4-dichloropyridin-3-yl, 2,4,6-trifluoropyridin-3-yl, 2,4,6-trichloropyridin-3-yl, 3,5-difluoropyridin-4-yl, 3,5-dichloropyridin-4-yl, 2,5-difluorothiophen-3-yl and 2,5-dichlorothiophen-3-yl, 3-trifluoromethylpyridin-2-yl, 5-chloropyrimidin-4-yl and, where appropriate, N-oxides thereof.

In the above definition halogen is fluorine, chlorine, bromine or iodine.

The alkyl, alkenyl or alkynyl radicals may be straight-chained or branched.

Alkyl on its own or as part of another substituent is, depending upon the number of carbon atoms mentioned, for example, methyl, ethyl, propyl, butyl, pentyl, hexyl and the isomers thereof, for example, isopropyl, isobutyl, sec-butyl, tert-butyl, isopentyl or tert-pentyl.

A haloalkyl group may contain one or more identical or different halogen atoms and, for example, may stand for CH₂Cl, CHCl₂, CCl₃, CH₂F, CHF₂, CF₃, CF₃CH₂, CH₃CF₂, CF₃CF₂, or CCl₃CCl₂.

Cycloalkyl on its own or as part of another substituent is, depending upon the number of carbon atoms mentioned, for example, cyclopropyl, cyclobutyl, cyclopentyl or cyclohexyl.

Alkenyl on its own or as part of another substituent is, depending upon the number of carbon atoms mentioned, for example, ethenyl, allyl, 1-propenyl, buten-2-yl, buten-3-yl, penten-1-yl, penten-3-yl, hexen-1-yl or 4-methyl-3-pentenyl.

Alkynyl on its own or as part of another substituent is, depending upon the number of carbon atoms mentioned, for example, ethynyl, propyn-1-yl, propyn-2-yl, butyn-1-yl, butyn-2-yl, 1-methyl-2-butynyl, hexyn-1-yl or 1-ethyl-2-butynyl.

The presence of one or more possible asymmetric carbon atoms in compounds of formula I means that the compounds may occur in optically isomeric, that means enantiomeric or diastereomeric forms. As a result of the presence of a possible aliphatic C=C double bond, geometric isomerism, that means cis-trans or (*E*)-(*Z*) isomerism may also occur. Also atropisomers may occur as a result of restricted rotation about a single bond. Formula I is intended to include all those possible isomeric forms and mixtures thereof. The present invention intends to include all those possible isomeric forms and mixtures thereof for a compound of formula I.

In each case, the compounds of formula I according to the invention are in free form or in an agronomically usable salt form.

Preferred sub-groups of compounds of formula I according to the invention are those wherein
X is N⁺-O⁻ and Y is N; or
X is N and Y is N⁺-O⁻; or
X and Y are both N⁺-O⁻;
R is H, halo, cyano, C₁₋₄ alkyl or C₁₋₄alkoxy;
R¹ is aryl or heteroaryl;
R² is C₁₋₈ alkyl, C₃₋₈ cycloalkyl or NR³R⁴;
R³ and R⁴ independently of each other are H, C₁₋₈ alkyl, halo(C₁₋₈)alkyl, C₂₋₈ alkenyl, C₂₋₈ alkynyl, aryl, aryl(C₁₋₈)alkyl, C₃₋₈ cycloalkyl, C₃₋₈ cycloalkyl(C₁₋₆)alkyl, heteroaryl, heteroaryl(C₁₋₈)alkyl or NR⁵R⁶, provided that not both R³ and R⁴ are NR⁵R⁶; or
R³ and R⁴ together with the nitrogen to which they are attached form a C₃₋₁₀ alkylene chain or a C₃₋₇ alkenylene chain optionally substituted with halo, C₁₋₄ alkyl, C₁₋₄ haloalkyl or C₁₋₄ alkoxy; or
R³ and R⁴ together with the nitrogen atom to which they are attached form a morpholine, thiomorpholine, thiomorpholine S-oxide or thiomorpholine S-dioxide ring, a piperazine or piperazine *N*-(C₁₋₄)alkyl (especially *N*-methyl) ring; or
R³ and R⁴ together with the nitrogen to which they are attached form a bicyclic annelated or bridged ring;
R⁵ and R⁶ independently of each other are H, C₁₋₈ alkyl, C₂₋₈ alkenyl, C₂₋₈ alkynyl, aryl or C₃₋₈ cycloalkyl;
R⁷ and R⁸ independently of each other are H, halo, cyano, C₁₋₄alkyl, C₁₋₄alkoxy, halo(C₁₋₄)alkyl, C₁₋₄alkylthio, C₁₋₄alkylsulphinyl, C₁₋₄alkylsulphonyl or NR⁹R¹⁰
R⁹ and R¹⁰ independently of each other are H or C₁₋₈ alkyl; or
R⁹ and R¹⁰ together with the nitrogen atom to which they are attached form a C₃₋₁₀ alkylene chain.

More preferred subgroups of compounds of formula I according to the invention are those wherein
X is N⁺-O⁻and Y is N; or
X is N and Y is N⁺-O⁻;
R is H, halo, cyano, C₁₋₄ alkyl or C₁₋₄ alkoxy;
R¹ is aryl or heteroaryl;
R² is C₁₋₈ alkyl or NR³R⁴;
R³ and R⁴ independently of each other are H, C₁₋₈ alkyl, halo(C₁₋₈)alkyl, C₂₋₈ alkenyl, C₂₋₈ alkynyl, C₃₋₈ cycloalkyl or C₃₋₈ cycloalkyl(C₁₋₆)alkyl;
R⁷ and R⁸ independently of each other are H, halo, cyano, C₁₋₄alkyl, C₁₋₄alkoxy or halo(C₁₋₄)alkyl.

Most preferred subgroups of compounds of formula I according to the invention are those wherein
X is N and Y is N⁺-O⁻;
R is halo, C₁₋₄ alkyl or C₁₋₄alkoxy;
R¹ is phenyl substituted in at least one ortho position with halogen;
R² is NR³R⁴;
R³ and R⁴ independently of each other are H, C₁₋₈ alkyl, halo(C₁₋₈)alkyl, C₂₋₈ alkenyl, C₂₋₈ alkynyl, C₃₋₈ cycloalkyl or C₃₋₈ cycloalkyl(C₁₋₆)alkyl;
R⁷ and R⁸ are H.

Especially preferred subgroups of compounds of formula I according to the invention are those wherein
X is N and Y is N⁺-O⁻;
R is chlorine or fluorine;
R¹ is 2,4,6-trifluorophenyl;
R² is NR³R⁴;
R³ is H;
R⁴ is C₁₋₈alkyl or halo(C₁₋₈)alkyl;
R⁷ and R⁸ are H.

Particularly preferred subgroups of compounds of formula I according to the invention are those wherein
X is N and Y is N⁺-O⁻;
R is chlorine or fluorine;
R¹ is 2,4,6-trifluorophenyl;
R² is NR³R⁴;
R³ is H;
R⁴ is C₁₋₈ alkyl or fluoro(C₁₋₈)alkyl;
R⁷ and R⁸ are H.

In the above definition of preferred subgroups of compounds of formula I,
any of the foregoing alkyl, alkenyl, alkynyl or cycloalkyl groups or moieties (other than for R⁷ and R⁸, independently of each other) are being optionally substituted with halogen, cyano, C₁₋₆ alkoxy, C₁₋₆ alkylcarbonyl, C₁₋₆ alkoxycarbonyl, C₁₋₆ haloalkoxy, C₁₋₆ alkylthio, tri(C₁₋₄)alkylsilyl, C₁₋₆ alkylamino or C₁₋₆ dialkylamino;
any of the foregoing morpholine, thiomorpholine, piperidine, piperazine and pyrrolidine rings are being optionally substituted with C₁₋₄ alkyl (especially methyl); and
any of the foregoing aryl or heteroaryl groups or moieties are being optionally substituted with one or more substituents selected from halo, hydroxy, mercapto, C₁₋₆ alkyl, C₂₋₆ alkenyl, C₂₋₆ alkynyl, C₁₋₆ alkoxy, C₂₋₆ alkenyloxy, C₂₋₆ alkynyloxy, halo(C₁₋₆)alkyl, halo(C₁₋₆)alkoxy, C₁₋₆ alkylthio, halo(C₁₋₆)alkylthio, hydroxy(C₁₋₆)alkyl C₁₋₄alkoxy(C₁₋₆)alkyl, C₃₋₆ cycloalkyl, C₃₋₆ cycloalkyl(C₁₋₄)alkyl, phenoxy, benzyloxy, benzoyloxy, cyano, isocyano, thiocyanato, isothiocyanato, nitro, -NR"'R"", -NHCOR"', -NHCONR"'R"", -CONR"'R"", - SO₂R"', -OSO₂R"', -COR"', -CR"'=NR"" or -N=CR"'R"", in which R"' and R"" are independently hydrogen, C₁₋₄ alkyl, halo(C₁₋₄)alkyl, C₁₋₄ alkoxy, halo(C₁₋₄)alkoxy, C₁₋₄ alkylthio, C₃₋₆ cycloalkyl, C₃₋₆ cycloalkyl(C₁₋₄)alkyl, phenyl or benzyl, the phenyl and benzyl groups being optionally substituted with halogen, C₁₋₄ alkyl or C₁₋₄ alkoxy.

Preferred individual compounds are:
tert-butyl-[6-fluoro-4-oxy-7-(2,4,6-trifluorophenyl)-pyrido[2,3-b]pyrazin-8-yl]-amine (Compound N° I.a.006),
[6-fluoro-4-oxy-7-(2,4,6-trifluorophenyl)-pyrido[2,3-b]pyrazin-8-yl]-(2,2,2-trifluoro-1-methylethyl)-amine (Compound N° I.a.008),
(S)-[6-fluoro-4-oxy-7-(2,4,6-tdfluorophenyl)-pyrido[2,3-b]pyrazin-8-yl]-(2,2,2-trifluoro-1-methylethyl)-amine (Compound N° I.a.009),
[6-fluoro-4-oxy-7-(2,4,6-trifluorophenyl)-pyrido[2,3-b]pyrazin-8-yl]-(1,2-dimethylpropyl)-amine (Compound N° I.a.021),
(R)- [6-fluoro-4-oxy-7-(2,4,6-trifluorophenyl)-pyrido[2,3-b]pyrazin-8-yl]-(1,2-dimethyl-propyl)-amine (Compound N° I.a.023),
[6-fluoro-4-oxy-7-(2,4,6-trifluorophenyl)-pyrido[2,3-b]pyrazin-8-yl]-(1,2,2-trimethyl-propyl)-amine (Compound N° I.a.024),
(R)- [6-fluoro-4-oxy-7-(2,4,6-trifluorophenyl)-pyrido[2,3-b]pyrazin-8-yl]-(1,2,2-trimethylpropyl)-amine (Compound N° I.a.026),
[6-fluoro-4-oxy-7-(2,4,6-trifluorophenyl)-pyrido[2,3-b]pyrazin-8-yl]-(3,3,3-trifluoro-1-methylpropyl)-amine (Compound N° I.a.045),
[7-(2,4-dichloro-phenyl)-6-fluoro-4-oxy-pyrido[2,3-b]pyrazin-8-yl]-(1,2-dimethylpropyl)-amine (Compound N° l.b.021),
[7-(2,4-dichloro-phenyl)-6-fluoro-4-oxy-pyrido[2,3-b]pyrazin-8-yl]-(1,2,2-trimethyl-propyl)-amine (Compound N° l.b.024),
[6-chloro-4-oxy-7-(2,4,6-trifluorophenyl)-pyrido[2,3-b]pyrazin-8-yl]-(1,2-dimethylpropyl)-amine (Compound N° l.g.021),
[6-chloro-4-oxy-7-(2,4,6-trifluorophenyl)-pyrido[2,3-b]pyrazin-8-yl]-(1,2,2-trimethyl-propyl)-amine (Compound N° l.g.024).

Compounds of formula (II), (III) and (IV), which are examples of compounds of general formula (I) can be made as shown in Scheme 1, in which R, R¹, R², R⁷ and R⁸ have the meanings given above.

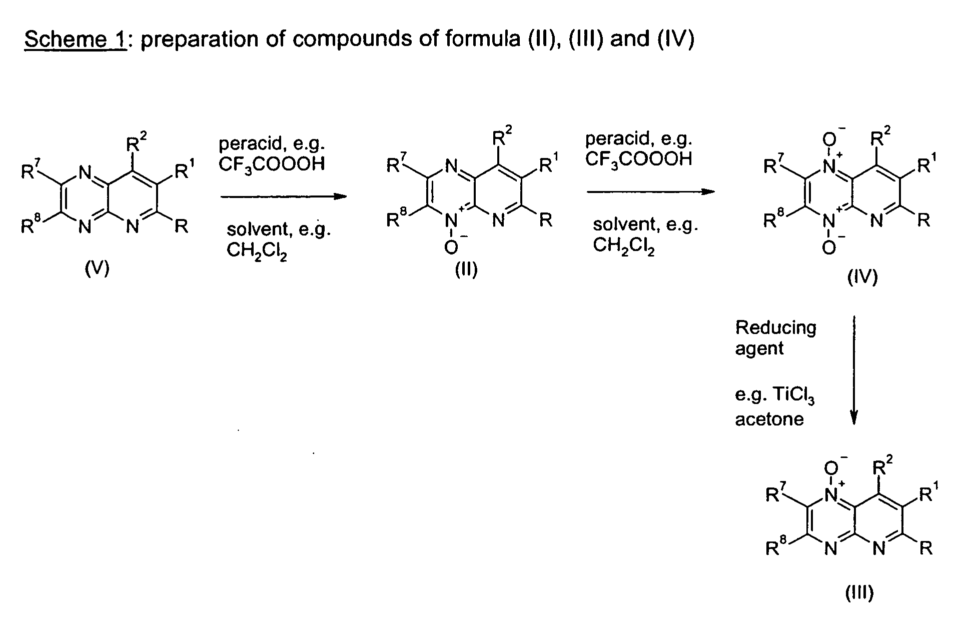

Compounds of general formula (V) can be prepared according to procedures described in WO04056825.

Compounds of general formula (II) can be prepared by reaction of compounds of general formula (V) with a percarboxylic acid (peracide), for example pertrifluoroacetic acid, generated by reaction of hydrogen peroxide, optionally on solid urea pellets, with trifluoroacetic acid in suitable solvent such as dichloromethane at room temperature.

Compounds of general formula (IV) can be prepared by reaction of compounds of general formula (II) with a percarboxylic acid (peracide), for example pertrifluoroacetic acid, generated by reaction of hydrogen peroxide, optionally on solid urea pellets, with trifluoroacetic acid in suitable solvent such as dichloromethane at room temperature.

Compounds of general formula (III) can be prepared from compounds of formula (IV) by reduction with a reducing agent such as titanium trichloride in methanol at 0°C to room temperature.

Surprisingly, it has now been found that the novel compounds of formula I have, for practical purposes, a very advantageous spectrum of activities for protecting plants against diseases that are caused by fungi as well as by bacteria and viruses.

The compounds of formula I can be used in the agricultural sector and related fields of use as active ingredients for controlling plant pests or on non-living materials for control of spoilage microorganisms or organisms potentially harmful to man. The novel compounds are distinguished by excellent activity at low rates of application, by being well tolerated by plants and by being environmentally safe. They have very useful curative, preventive and systemic properties and are used for protecting numerous cultivated plants. The compounds of formula I can be used to inhibit or destroy the pests that occur on plants or parts of plants (fruit, blossoms, leaves, stems, tubers, roots) of different crops of useful plants, while at the same time protecting also those parts of the plants that grow later e.g. from phytopathogenic microorganisms.

It is also possible to use compounds of formula I as dressing agents for the treatment of plant propagation material, *e.g.,* seed, such as fruits, tubers or grains, or plant cuttings (for example rice), for the protection against fungal infections as well as against phytopathogenic fungi occurring in the soil. The propagation material can be treated with a composition comprising a compound of formula I before planting: seed, for example, can be dressed before being sown. The active ingredients according to the invention can also be applied to grains (coating), either by impregnating the seeds in a liquid formulation or by coating them with a solid formulation. The composition can also be applied to the planting site when the propagation material is being planted, for example, to the seed furrow during sowing. The invention relates also to such methods of treating plant propagation material and to the plant propagation material so treated.

Furthermore the compounds according to present invention can be used for controlling fungi in related areas, for example in the protection of technical materials, including wood and wood related technical products, in food storage, in hygiene management.

In addition, the invention could be used to protect non-living materials from fungal attack, e.g. lumber, wall boards and paint.

The compounds of formula I are, for example, effective against the phytopathogenic fungi of the following classes: Fungi imperfecti (e.g. *Botrytis* spp., *Alternaria* spp.) and Basidiomycetes (e.g. *Rhizoctonia* spp., *Hemileia* spp., *Puccinia* spp., *Phakopsora* spp., *Ustilago spp., Tilletia spp.*). Additionally, they are also effective against Ascomycetes (e.g. *Venturia* spp., *Blumeria* spp., *Podosphaera leucotricha, Monilinia* spp., *Fusarium* spp., *Uncinula* spp., *Mycosphaerella* spp., *Pyrenophora* spp., *Rhynchosporium* secalis, *Magnaporthe* spp., *Colletotrichum* spp., *Gaeumannomyces graminis, Tapesia spp., Ramularia spp., Microdochium nivale, Sclerotinia spp.)* and Oomycetes (e.g. *Phytophthora* spp., *Pythium* spp., *Plasmopara* spp., *Pseudoperonospora cubensis).* Outstanding activity has been observed against powdery mildews (e.g. *Uncinula necator),* rusts (e.g. *Puccinia* spp.) and leaf spots (e.g. *Septoria tritici).* Furthermore, the novel compounds of formula I are effective against phytopathogenic bacteria and viruses (e.g. against *Xanthomonas* spp, *Pseudomonas* spp, *Erwinia amylovora* as well as against the tobacco mosaic virus).

Within the scope of present invention, target crops to be protected typically comprise the following species of plants: cereal (wheat, barley, rye, oat, rice, maize, sorghum and related species); beet (sugar beet and fodder beet); pomes, drupes and soft fruit (apples, pears, plums, peaches, almonds, cherries, strawberries, raspberries and blackberries); leguminous plants (beans, lentils, peas, soybeans); oil plants (rape, mustard, poppy, olives, sunflowers, coconut, castor oil plants, cocoa beans, groundnuts); cucumber plants (pumpkins, cucumbers, melons); fibre plants (cotton, flax, hemp, jute); citrus fruit (oranges, lemons, grapefruit, mandarins); vegetables (spinach, lettuce, asparagus, cabbages, carrots, onions, tomatoes, potatoes, paprika); lauraceae (avocado, cinnamomum, camphor) or plants such as tobacco, nuts, coffee, eggplants, sugar cane, tea, pepper, vines, hops, bananas and natural rubber plants, as well as turf and ornamentals.

The target crops in accordance with the invention include conventional as well as genetically enhanced or engineered varieties such as, for example, insect resistant (e.g. Bt. and VIP varieties) as well as disease resistant, herbicide tolerant (e.g. glyphosate- and glufosinate-resistant maize varieties commercially available under the trade names RoundupReady® and LibertyLink®) and nematode tolerant varieties. By way of example, suitable genetically enhanced or engineered crop varieties include the Stoneville 5599BR cotton and Stoneville 4892BR cotton varieties.

The compounds of formula I are used in unmodified form or, preferably, together with the adjuvants conventionally employed in the art of formulation. To this end they are conveniently formulated in known manner to emulsifiable concentrates, coatable pastes, directly sprayable or dilutable solutions or suspensions, dilute emulsions, wettable powders, soluble powders, dusts, granulates, and also encapsulations e.g. in polymeric substances. As with the type of the compositions, the methods of application, such as spraying, atomising, dusting, scattering, coating or pouring, are chosen in accordance with the intended objectives and the prevailing circumstances. The compositions may also contain further adjuvants such as stabilizers, antifoams, viscosity regulators, binders or tackifiers as well as fertilizers, micronutrient donors or other formulations for obtaining special effects.

Suitable carriers and adjuvants can be solid or liquid and are substances useful in formulation technology, e.g. natural or regenerated mineral substances, solvents, dispersants, wetting agents, tackifiers, thickeners, binders or fertilizers. Such carriers are for example described in WO 97/33890.

The compounds of formula I are normally used in the form of compositions and can be applied to the crop area or plant to be treated, simultaneously or in succession with further compounds. These further compounds can be e.g. fertilizers or micronutrient donors or other preparations, which influence the growth of plants. They can also be selective herbicides as well as insecticides, fungicides, bactericides, nematicides, molluscicides or mixtures of several of these preparations, if desired together with further carriers, surfactants or application promoting adjuvants customarily employed in the art of formulation.

The compounds of formula I are normally used in the form of fungicidal compositions for controlling or protecting against phytopathogenic microorganisms, comprising as active ingredient at least one compound of formula I, in free form or in agrochemically usable salt form, and at least one of the above-mentioned adjuvants.

The compounds of formula I can be mixed with other fungicides, resulting in some cases in unexpected synergistic activities. Mixing components which are particularly preferred are:
Azoles, such as azaconazole, BAY 14120, bitertanol, bromuconazole, cyproconazole, difenoconazole, diniconazole, epoxiconazole, fenbuconazole, fluquinconazole, flusilazole, flutriafol, hexaconazole, imazalil, imibenconazole, ipconazole, metconazole, myclobutanil, pefurazoate, penconazole, prothioconazole, pyrifenox, prochloraz, propiconazole, simeconazole, tebuconazole, tetraconazole, triadimefon, triadimenol, triflumizole, triticonazole;
Pyrimidinyl carbinoles, such as ancymidol, fenarimol, nuarimol;
2-amino-pyrimidines, such as bupirimate, dimethirimol, ethirimol;
Morpholines, such as dodemorph, fenpropidine, fenpropimorph, spiroxamine, tridemorph;
Anilinopyrimidines, such as cyprodinil, mepanipyrim, pyrimethanil;
Pyrroles, such as fenpiclonil, fludioxonil;
Phenylamides, such as benalaxyl, furalaxyl, metalaxyl, R-metalaxyl, ofurace, oxadixyl;
Benzimidazoles, such as benomyl, carbendazim, debacarb, fuberidazole, thiabendazole;
Dicarboximides, such as chlozolinate, dichlozoline, iprodione, myclozoline, procymidone, vinclozoline;
Carboxamides, such as boscalid, carboxin, fenfuram, flutolanil, mepronil, oxycarboxin, penthiopyrad, thifluzamide; guanidines, such as guazatine, dodine, iminoctadine;
Strobilurines, such as azoxystrobin, dimoxystrobin, enestroburin, fluoxastrobin, kresoxim-methyl, metominostrobin, trifloxystrobin, orysastrobin, picoxystrobin, pyraclostrobin;
Dithiocarbamates, such as ferbam, mancozeb, maneb, metiram, propineb, thiram, zineb, ziram;
N-halomethylthiotetrahydrophthalimides, such as captafol, captan, dichlofluanid, fluoromides, folpet, tolyfluanid;
Cu-compounds, such as Bordeaux mixture, copper hydroxide, copper oxychloride, copper sulfate, cuprous oxide, mancopper, oxine-copper;
Nitrophenol-derivatives, such as dinocap, nitrothal-isopropyl;
Organo-p-derivatives, such as edifenphos, iprobenphos, isoprothiolane, phosdiphen, pyrazophos, tolclofos-methyl;
Pyridazine-derivatives which are known and may be prepared by methods as described in WO 05/121104 and WO 06/001175, such as 3-chloro-5-(4-chloro-phenyl)-6-methyl-4-(2,4,6-trifluoro-phenyl)-pyridazine (formula P.1) and 3-chloro-6-methyl-5-p-tolyl-4-(2,4,6-trifluoro-phenyl)-pyridazine (formula P.2);

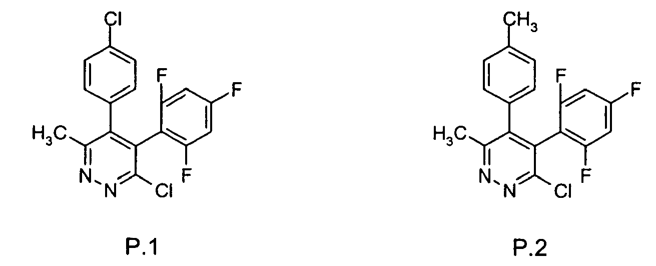

Various others, such as acibenzolar-S-methyl, anilazine, benthiavalicarb, blasticidin-S, chinomethionate, chloroneb, chlorothalonil, cyflufenamid, cymoxanil, dichlone, diclocymet, diclomezine, dicloran, diethofencarb, dimethomorph, flumorph, dithianon, ethaboxam, etridiazole, famoxadone, fenamidone, fenoxanil, fentin, ferimzone, fluazinam, fluopicolide, flusulfamide, fenhexamid, fosetyl-aluminium, hymexazol, iprovalicarb, cyazofamid, kasugamycin, mandipropamid, methasulfocarb, metrafenone, nicobifen, pencycuron, phthalide, polyoxins, probenazole, propamocarb, proquinazid, pyroquilon, quinoxyfen, quintozene, sulfur, tiadinil, triazoxide, tricyclazole, triforine, validamycin, zoxamide.

A method of controlling or preventing an infestation of crop plants, harvested food crops or of non-living materials by phytopathogenic or spoilage microorganisms or organisms potentially harmful to man, especially fungal organisms, which comprises the application of a compound of formula I as active ingredient to the plants, to parts of the plants or to the locus thereof, to harvested food crops, to seeds or to any part of the non-living materials. Controlling or preventing means reducing the infestation of crop plants or of non-living materials by phytopathogenic or spoilage microorganisms or organisms potentially harmful to man, especially fungal organisms, to such a level that an improvement is demonstrated.

A preferred method of controlling or preventing an infestation of crop plants by phytopathogenic microorganisms, especially fungal organisms, which comprises the application of a compound of formula I, or an agrochemical composition which contains at least one of said compounds, is foliar application. The frequency of application and the rate of application will depend on the risk of infestation by the corresponding pathogen. However, the compounds of formula I can also penetrate the plant through the roots via the soil (systemic action) by drenching the locus of the plant with a liquid formulation, or by applying the compounds in solid form to the soil, e.g. in granular form (soil application). In crops of water rice such granulates can be applied to the flooded rice field. The compounds of formula I may also be applied to seeds (coating) by impregnating the seeds or tubers either with a liquid formulation of the fungicide or coating them with a solid formulation.

A formulation [that is, a composition containing the compound of formula I] and, if desired, a solid or liquid adjuvant or monomers for encapsulating the compound of formula I, is prepared in a known manner, typically by intimately mixing and/or grinding the compound with extenders, for example solvents, solid carriers and, optionally, surface active compounds (surfactants).

The agrochemical formulations will usually contain from 0.1 to 99% by weight, preferably from 0.1 to 95% by weight, of the compound of formula 1, 99.9 to 1 % by weight, preferably 99.8 to 5% by weight, of a solid or liquid adjuvant, and from 0 to 25% by weight, preferably from 0.1 to 25% by weight, of a surfactant.
Advantageous rates of application are normally from 5g to 2kg of active ingredient (a.i.) per hectare (ha), preferably from 10g to 1kg a.i./ha, most preferably from 20g to 600g a.i./ha. When used as seed drenching agent, convenient dosages are from 10mg to 1g of active ingredient per kg of seeds.

Whereas it is preferred to formulate commercial products as concentrates, the end user will normally use dilute formulations.

The following non-limiting Examples illustrate the above-described invention in more detail.

### Example 1: This Example illustrates the preparation of (R)-[6-fluoro-4-oxy-7-(2,4,6-trifluorophenyl)-pyrido[2,3-b]pyrazin-8-yl]-(1,2-dimethylpropyl)-amine (Compound N°.I.a.023).

(R)-1,2-Dimethyl-propyl)-[6-fluoro-7-(2,4,6-trifluorophenyl)-pyrido[2,3-b]pyrazin-8-yl]-amine (0.25 g ) and urea hydrogen peroxide complex (0.099 g) are stirred in dichloromethane (5mL) under nitrogen, and the reaction cooled to 0 °C. Trifluoroacetic anhydride (0.203 g) is added dropwise via a micro-syringe. After 24 hours, the reaction mixture is quenched with aqueous sodium bisulphite (5mL), diluted with dichloromethane (10mL) and separated. The organic fraction is washed until all traces of peroxides are gone and the aqueous fraction is diluted until no peroxide remained and the combined organics washed with dilute hydrochloric acid (1 M) and brine. The organics are dried over magnesium sulphate and concentrated *in vacuo* to give a pale cream foam, which is purified by flash column chromatography on silica gel, eluting with ethyl acetate : hexane, 1:1 to give the title product (0.070 g).

Table 1 below illustrates examples of individual compounds of formula I according to the invention.

**Table 1: individual compounds of formula I according to the invention**

| Compound N° | R³ | R⁴ | | Compound N° | R³ | R⁴ |
|---|---|---|---|---|---|---|
| 001 | CH₃ | H | | 033 | (R)-CH₃CH₂(CF₃)CH | H |
| 002 | C₂H₅ | H | | 034 | (CH₃)₂CH(CF₃)CH | H |
| 003 | *n*-C₃H₇ | H | | 035 | (S)-(CH₃)₂CH(CF₃)CH | H |
| 004 | *i*-C₃H₇ | H | | 036 | (R)-(CH₃)₂CH(CF₃)CH | H |
| 005 | *n*-C₄H₉ | H | | 037 | (CH₃)₃C(CF₃)CH | H |
| 006 | *t*-C₄H₉ | H | | 038 | (S)-(CH₃)₃C(CF₃)CH | H |
| 007 | CF₃CH₂ | H | | 039 | (R)-(CH₃)₃C(CF₃)CH | H |
| 008 | CF₃(CH₃)CH | H | | 040 | (cyclopropyl)(CH₃)CH | H |
| 009 | (S)-CF₃(CH₃)CH | H | | 041 | (2,2-diF-cyclopropyl) (CH₃)CH | H |
| 010 | (R)-CF₃(CH₃)CH | H | | | | |
| 011 | Cyclopropyl | H | | 042 | (cyclopropyl)(CF₃)CH | H |
| 012 | Cyclobutyl | H | | 043 | (S)-(cyclopropyl)(CF₃)CH | H |
| 013 | Cyclopentyl | H | | | | |
| 014 | Cyclopropyl-CH₂ | H | | 044 | (R)-(cyclopropyl)(CF₃)CH | H |
| 015 | Cyclobutyl-CH₂ | H | | | | |
| 016 | -(CH₂)₂O(CH₂)₂- | | | 045 | CF₃CH₂(CH₃)CH | H |
| 017 | -(CH₂)₂CH(CH₃)(CH₂)₂- | | | 046 | CF₃(CH₃)CH(CH₃)CH | H |
| 018 | CH₃CH₂(CH₃)CH | H | | 047 | Bicyclo[2.2.1]hept-2-yl | H |
| 019 | (S)-CH₃CH₂(CH₃)CH | H | | 048 | 1-CH₃-cyclopropyl | H |
| 020 | (R)-CH₃CH₂(CH₃)CH | H | | 049 | *cis*-2-CH₃-cyclopropyl | H |
| 021 | (CH₃)₂CH(CH₃)CH | H | | 050 | *trans*-2-CH₃-cyclopropyl | H |
| 022 | (S)-(CH₃)₂CH(CH₃)CH | H | | | | |
| 023 | (R)-(CH₃)₂CH(CH₃)CH | H | | 051 | 2,2-(CH₃)₂-cyclopropyl | H |
| 024 | (CH₃)₃C(CH₃)CH | H | | 052 | 1-CH₃-cyclobutyl | H |
| 025 | (S)-(CH₃)₃C(CH₃)CH | H | | 053 | *cis*-2-CH₃-cyclobutyl | H |
| 026 | (R)-(CH₃)₃C(CH₃)CH | H | | 054 | *trans*-2-CH₃-cyclobutyl | H |
| 027 | (CH₃)₂CHCH₂ | H | | 055 | *cis*-3-CH₃-cyclobutyl | H |
| 028 | (CH₃)₃CCH₂ | H | | 056 | *trans*-3-CH₃-cyclobutyl | H |
| 029 | (CH₃)₂CH | H | | 057 | 2,2-(CH₃)₂-cyclobutyl | H |
| 030 | CH₃CH₂(CH₃)₂C | H | | 058 | 3,3-(CH₃)₂-cyclobutyl | H |
| 031 | CH₃CH₂(CF₃)CH | H | | 059 | 1-CH₃-cyclopentyl | H |
| 032 | (S)-CH₃CH₂(CF₃)CH | H | | 060 | -(CH₂)₄- | |
| 061 | -(CH₂)₅- | | | 080 | CH₃CH₂(CH₃)₂C | CH₃ |
| 062 | -(CH₂)₂(CH₃)₂C(CH₂)₂- | | | 081 | CH₃CH₂(CF₃)CH | CH₃ |
| 063 | -(CH₃)CH(CH₂)₂- | | | 082 | (CH₃)₂CH(CF₃)CH | CH₃ |
| 064 | -(CH₃)CH(CH₂)₃- | | | 083 | (CH₃)₃C(CF₃)CH | CH₃ |
| 065 | *n*-C₃H₇ | CH₃ | | 084 | CH₃CH₂CH₂(CF₃)CH | CH₃ |
| 066 | *i*-C₃H₇ | CH₃ | | 085 | (cyclopropyl)(CF₃)CH | CH₃ |
| 067 | *t*-C₄H₉ | CH₃ | | 086 | CF₃CH₂(CH₃)CH | CH₃ |
| 068 | CF₃CH₂ | CH₃ | | 087 | Bicyclo[2.2.1]hept-2-yl | CH₃ |
| 069 | CF₃(CH₃)CH | CH₃ | | 088 | 1-CH₃-cyclopropyl | CH₃ |
| 070 | (S)-CF₃(CH₃)CH | CH₃ | | 089 | 2-CH₃-cyclopropyl | CH₃ |
| 071 | (R)-CF₃(CH₃)CH | CH₃ | | 090 | 2,2-(CH₃)₂-cyclopropyl | CH₃ |
| 072 | cyclo-C₃H₅ | CH₃ | | 091 | 1-CH₃-cyclobutyl | CH₃ |
| 073 | cyclo-C₄H₇ | CH₃ | | 092 | 2-CH₃-cyclobutyl | CH₃ |
| 074 | CH₃CH₂(CH₃)CH | CH₃ | | 093 | 2,2-(CH₃)₂-cyclobutyl | CH₃ |
| 075 | (CH₃)₂CH(CH₃)CH | CH₃ | | 094 | 3,3-(CH₃)₂-cyclobutyl | CH₃ |
| 076 | (CH₃)₃C(CH₃)CH | CH₃ | | 095 | Cyclopentyl | CH₃ |
| 077 | (CH₃)₂CHCH₂ | CH₃ | | 096 | 1-CH₃-cyclopentyl | CH₃ |
| 078 | (CH₃)₃CCH₂ | CH₃ | | 097 | Cyclohexyl | H |
| 079 | (CH₃)CH | CH₃ | | 098 | 1-CH₃-cyclohexyl | H |

where
(a) 98 compounds of formula (I.a) 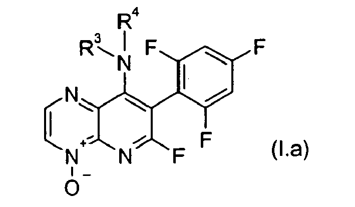 wherein R³ and R⁴ are as defined in Table 1.
(b) 98 compounds of formula (I.b) 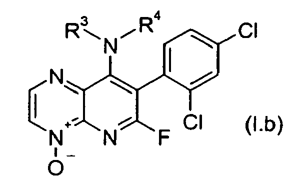 wherein R³ and R⁴ are as defined in Table 1.
(c) 98 compounds of formula (I.c) 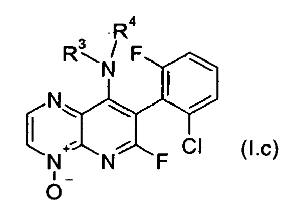 wherein R³ and R⁴ are as defined in Table 1.
(d) 98 compounds of formula (I.d) 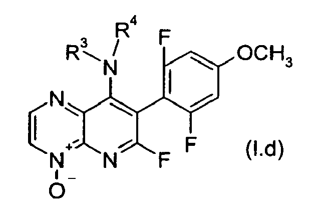 wherein R³ and R⁴ are as defined in Table 1.
(e) 98 compounds of formula (I.e) 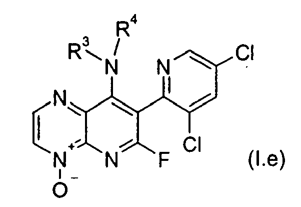 wherein R³ and R⁴ are as defined in Table 1.
(f) 98 compounds of formula (I.f) 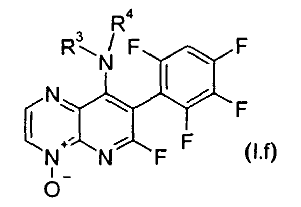 wherein R³ and R⁴ are as defined in Table 1.
(g) 98 compounds of formula (I.g) 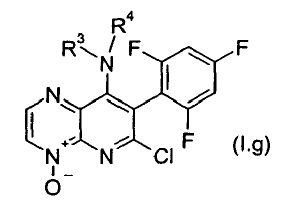 wherein R³ and R⁴ are as defined in Table 1.
(h) 98 compounds of formula (I.h) 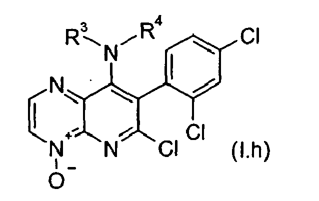 wherein R³ and R⁴ are as defined in Table 1.
(i) 98 compounds of formula (I.i) 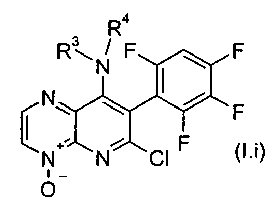 wherein R³ and R⁴ are as defined in Table 1.

Throughout this description, temperatures are given in degrees Celsius; "NMR" means nuclear magnetic resonance spectrum; and "%" is percent by weight, unless corresponding concentrations are indicated in other units.

The following abbreviations are used throughout this description:

| | | | |
|---|---|---|---|
| m.p. = | melting point | br = | broad |
| s = | singlet | dd = | doublet of doublets |
| d = | doublet | dt = | doublet of triplets |
| t = | triplet | q = | quartet |
| m = | multiplet | ppm = | parts per million |

Table 2 shows selected NMR data, all with CDCl₃ as the solvent (unless otherwise stated, no attempt is made to list all characterising data in all cases) for compounds of Table 1.

**Table 2: NMR data for selected compounds of Table 1**

| Compound Number | ¹H-NMR data (ppm/ number of Hs/multiplicity) |
|---|---|
| I.a.023 | 0.80 (6H, t), 1.05 (3H, d), 1.65 (1H, m), 3.08 (1H, m), 6.85 (2H, t), 7.32 (1H, d), 8.40 (1H, d), 8.52 (1H, d). |
| I.a.026 | 0.84 (9H, s), 0.98 (3H, d), 3.04 (1H, m), 6.82 (2H, t), 7.42 (1H, d), 8.38 (1H, d), 8.52 (1H, d) |
| l.a.045 | 1.10 (3H, d), 2.35 (1H, m), 3.12 (1H, m), 3.35 (1H, m), 6.86 (2H, m), 7. 40 (1H, br. t), 8.42 (1H, d), 8.50 (1H, d) |

The compounds according to the present invention can be prepared according to the above-mentioned reaction schemes, in which, unless otherwise stated, the definition of each variable is as defined above for a compound of formula (I).

### Biological examples

### Altemaria solani / tomato / preventive (Action against Alternaria on tomato)

4 weeks old tomato plants cv. Roter Gnom are treated with the formulated test compound in a spray chamber. Two days after application tomato plants are inoculated by spraying a spore suspension on the test plants. After an incubation period of 4 days at 22/18° C and 95% relative humidity in a greenhouse the disease incidence is assessed.

Compounds of formula I according to the invention, in particular compounds I.a.023 and I.a.026 at 200 ppm inhibit fungal infestation in this test to at least 80 %, while under the same conditions untreated control plants are infected by the phytopathogenic fungi to over 80%.

### Botrytis cinerea / tomato / preventive (Action against Botrytis on tomato)

4 weeks old tomato plants cv. Roter Gnom are treated with the formulated test compound in a spray chamber. Two days after application tomato plants are inoculated by spraying a spore suspension on the test plants. After an incubation period of 3 days at 20° C and 95% relative humidity in a greenhouse the disease incidence is assessed.

Compounds of formula I according to the invention, in particular compounds I.a.023 and I.a.026 at 200 ppm inhibit fungal infestation in this test to at least 80 %, while under the same conditions untreated control plants are infected by the phytopathogenic fungi to over 80 %.

### Puccinia recondita /wheat / preventive (Action against brown rust on wheat)

1 week old wheat plants cv. Arina are treated with the formulated test compound in a spray chamber. One day after application wheat plants are inoculated by spraying a spore suspension (1 x 105 uredospores/ml) on the test plants. After an incubation period of 1 day at 20° C and 95% relative humidity plants are kept for 10 days 20° C / 18° C (day/night) and 60% relative humidity in a greenhouse. The disease incidence is assessed 11 days after inoculation.

Compounds of formula I according to the invention, in particular compounds I.a.023 and I.a.026 at 200 ppm inhibit fungal infestation in this test to at least 80 %, while under the same conditions untreated control plants are infected by the phytopathogenic fungi to over 80 %.

### Pyrenophora teres (Helminthosporium teres) / barley / preventive (Action against net blotch on barley)

1-week-old barley plants cv. Regina are treated with the formulated test compound in a spray chamber. Two days after application barley plants are inoculated by spraying a spore suspension (2.6 x 10⁴ conidia/ml) on the test plants. After an incubation period of 4 days at 20° C and 95% relative humidity the disease incidence is assessed.

Compounds of formula I according to the invention, in particular compounds I.a.023 and I.a.026 at 200 ppm inhibit fungal infestation in this test to at least 80 %, while under the same conditions untreated control plants are infected by the phytopathogenic fungi to over 80 %.

### Septoria tritici / wheat / preventive (Action against Septoria leaf spot on wheat)

2 weeks old wheat plants cv. Riband are treated with the formulated test compound in a spray chamber. One day after application wheat plants are inoculated by spraying a spore suspension (10⁶ conidia/ml) on the test plants. After an incubation period of 1 day at 22°C/21°C and 95% relative humidity plants are kept at 22°C/21°C and 70% relative humidity in a greenhouse. The disease incidence is assessed 16 - 18 days after inoculation.

Compounds of formula I according to the invention, in particular compound I.a.023 at 200 ppm inhibits fungal infestation in this test to at least 80 %, while under the same conditions untreated control plants are infected by the phytopathogenic fungi to over 80 %.

### Uncinula necator / grape / preventive (Action against powdery mildew on grape)

5 weeks old grape seedlings cv. Gutedel are treated with the formulated test compound in a spray chamber. One day after application grape plants are inoculated by shaking plants infected with grape powdery mildew above the test plants. After an incubation period of 7 days at 24/22° C and 70% relative humidity under a light regime of 14/10 h (light/dark) the disease incidence is assessed.

Compounds of formula I according to the invention, in particular compound I.a.023 at 200 ppm inhibits fungal infestation in this test to at least 80 %, while under the same conditions untreated control plants are infected by the phytopathogenic fungi to over 80 %.

## Claims

1. A compound of formula I: 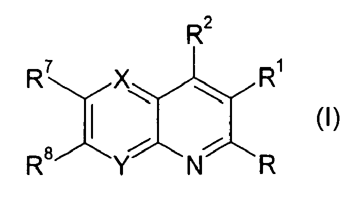 wherein
X is N⁺-O⁻and Y is N; or
X is N and Y is N⁺-O⁻; or
X and Y are both N⁺-O⁻;
R is H, halo, cyano, C₁₋₆ alkyl, C₁₋₆ alkoxy, C₁₋₆ alkylthio, halo(C₁₋₆)alkyl, C₁₋₆ alkenyl, C₁₋₆ alkynyl or NR³R⁴;
R¹ is an aryl or heteroaryl;
R² is halo, C₁₋₈ alkyl, C₃₋₈ cycloalkyl, aryl, heteroaryl, OR³, SR³ or NR³R⁴;
R³ and R⁴ independently of each other are H, C₁₋₈ alkyl, halo(C₁₋₈)alkyl, C₂₋₈ alkenyl, C₂₋₈ alkynyl, aryl, aryl(C₁₋₈)alkyl, C₃₋₈ cycloalkyl, C₃₋₈ cycloalkyl(C₁₋₆)alkyl, heteroaryl, heteroaryl(C₁₋₈)alkyl, NR⁵R⁶, provided that not both R³ and R⁴ are NR⁵R⁶; or
R³ and R⁴ together with the nitrogen atom to which they are attached form a C₃₋₁₀ alkylene chain or C₃₋₇ alkenylene chain optionally substituted with halo, C₁₋₄ alkyl, C₁₋₄ haloalkyl, C₁₋₄ alkoxy; or
R³ and R⁴ together with the nitrogen atom to which they are attached form a morpholine, thiomorpholine, thiomorpholine S-oxide or thiomorpholine S-dioxide ring or a piperazine or piperazine *N*-(C₁₋₄)alkyl ring; or
R³ and R⁴ together with the nitrogen atom to which they are attached form a bicyclic annelated or bridged ring;
R⁵ and R⁶ independently of each other are H, C₁₋₈ alkyl, C₂₋₈ alkenyl, C₂₋₈ alkynyl, aryl, aryl(C₁₋₈)alkyl, C₃₋₈ cycloalkyl, C₃₋₈ cycloalkyl(C₁₋₆)alkyl, heteroaryl or heteroaryl(C₁₋₈)alkyl;
R⁷ and R⁸ independently of each other are H, halo, cyano, C₁₋₄alkyl, C₁₋₄alkoxy, halo(C₁₋₄)alkyl, C₁₋₄alkylthio, C₁₋₄alkylsulphinyl, C₁₋₄alkylsulphonyl, aryl, heteroaryl, halo(C₁₋₆)alkoxy, halo(C₁₋₄)alkylthio, C₂₋₄alkenyl, C₂₋₆alkynyl, C₃₋₆cycloalkyl, C₁₋₆alkoxycarbonyl, R³R⁴NCO or NR⁹R¹⁰;
R⁹ and R¹⁰ independently of each other are H or C₁₋₈ alkyl; or
R⁹ and R¹⁰ together with the nitrogen atom to which they are attached form a C₃₋₁₀ alkylene chain; or
R⁹ and R¹⁰ together with the nitrogen atom to which they are attached form a morpholine, piperazine or piperazine *N*-(C₁₋₄)alkyl ring;
any of the foregoing alkyl, alkenyl, alkynyl or cycloalkyl groups or moieties (other than for R⁷ and R⁸, independently of each other) being optionally substituted with halogen, cyano, C₁₋₆ alkoxy, C₁₋₆ alkylcarbonyl, C₁₋₆ alkoxycarbonyl, C₁₋₆ haloalkoxy, C₁₋₆ alkylthio, tri(C₁₋₄)alkylsilyl, C₁₋₆ alkylamino or C₁₋₆ dialkylamino;
any of the foregoing morpholine, thiomorpholine, piperidine, piperazine and pyrrolidine rings being optionally substituted with C₁₋₄ alkyl (especially methyl); and
any of the foregoing aryl or heteroaryl groups or moieties being optionally substituted with one or more substituents selected from halo, hydroxy, mercapto, C₁₋₆ alkyl, C₂₋₆ alkenyl, C₂₋₆ alkynyl, C₁₋₆ alkoxy, C₂₋₆ alkenyloxy, C₂₋₆ alkynyloxy, halo(C₁₋₆)alkyl, halo(C₁₋₆)alkoxy, C₁₋₆ alkylthio, halo(C₁₋₆)alkylthio, hydroxy(C₁₋₆)alkyl, C₁₋₄alkoxy(C₁₋₆)alkyl, C₃₋₆ cycloalkyl, C₃₋₆ cycloalkyl(C₁₋₄)alkyl, phenoxy, benzyloxy, benzoyloxy, cyano, isocyano, thiocyanato, isothiocyanato, nitro, -NR"'R"", -NHCOR"', -NHCONR"'R"", -CONR"'R"", -SO₂R"', -OSO₂R"', -COR"', -CR"'=NR"" or -N=CR"'R"", in which R'" and R"" are independently hydrogen, C₁₋₄ alkyl, halo(C₁₋₄)alkyl, C₁₋₄ alkoxy, halo(C₁₋₄)alkoxy, C₁₋₄ alkylthio, C₃₋₆ cycloalkyl, C₃₋₆ cycloalkyl(C₁₋₄)alkyl, phenyl or benzyl, the phenyl and benzyl groups being optionally substituted with halogen, C₁₋₄ alkyl or C₁₋₄ alkoxy.

2. The compound according to claim 1 in free form.

3. The compound according to claim 1 in an agrochemically usable salt form.

4. The compound according to any one of claims 1 to 3 wherein
R is H, halo, cyano or C₁₋₄ alkyl, C₁₋₄ alkoxy;
R² is C₁₋₈ alkyl, C₃₋₈ cycloalkyl or NR³R⁴;
R⁵ and R⁶ independently of each other are H, C₁₋₈ alkyl, C₂₋₈ alkenyl, C₂₋₈ alkynyl, aryl or C₃₋₈ cycloalkyl;
R⁷ and R⁸ independently of each other are H, halo, cyano, C₁₋₄alkyl, C₁₋₄alkoxy,
halo(C₁₋₄)alkyl, C₁₋₄alkylthio, C₁₋₄alkylsulphinyl, C₁₋₄alkylsulphonyl or NR⁹R¹⁰.

5. The compound according to any one of claims 1 to 4 wherein
X is N⁺-O⁻and Y is N; or
X is N and Y is N⁺-O⁻;
R² is C₁₋₈ alkyl or NR³R⁴;
R³ and R⁴ independently of each other are H, C₁₋₈ alkyl, halo(C₁₋₈)alkyl, C₂₋₈ alkenyl, C₂₋₈ alkynyl, C₃₋₈ cycloalkyl or C₃₋₈ cycloalkyl(C₁₋₆)alkyl;
R⁷ and R⁸ independently of each other are H, halo, cyano, C₁₋₄alkyl, C₁₋₄alkoxy or halo(C₁₋₄)alkyl.

6. The compound according to any one of claims 1 to 5 wherein
X is N and Y is N⁺-O⁻;
R is halo, C₁₋₄ alkyl or C₁₋₄ alkoxy;
R¹ is phenyl substituted in at least one ortho position with halogen;
R² is NR³R⁴;
R⁷ and R⁸ are H.

7. The compound according to any one of claims 1 to 6 wherein
R is chlorine or fluorine;
R¹ is 2,4,6-trifluorophenyl;
R³ is H;
R⁴ is C₁₋₈ alkyl or halo(C₁₋₈)alkyl.

8. The compound of formula I according to any one of claims 1 to 7 wherein R⁴ is fluoro(C₁₋₈)alkyl.

9. A compound selected from:
tert-butyl-[6-fluoro-4-oxy-7-(2,4,6-trifluorophenyl)-pyrido[2,3-b]pyrazin-8-yl]-amine, [6-fluoro-4-oxy-7-(2,4,6-trifluorophenyl)-pyrido[2,3-b] pyrazin-8-yl]-(2,2,2-trifluoro-1-methylethyl)-amine,
(S)-[6-fluoro-4-oxy-7-(2,4,6-trifluorophenyl)-pyrido[2,3-b]pyrazin-8-yl]-(2,2,2-trifluoro-1-methylethyl)-amine,
[6-fluoro-4-oxy-7-(2,4,6-trifluorophenyl)-pyrido[2,3-b]pyrazin-8-yl]-(1,2-dimethylpropyl)-amine,
(R)- [6-fluoro-4-oxy-7-(2,4,6-trifluorophenyl)-pyrido[2,3-b]pyrazin-8-yl]-(1,2-dimethyl-propyl)-amine,
[6-fluoro-4-oxy-7-(2,4,6-trifluorophenyl)-pyrido[2,3-b]pyrazin-8-yl]-(1,2,2-trimethyl-propyl)-amine,
(R)- [6-fluoro-4-oxy-7-(2,4,6-trifluorophenyl)-pyrido[2,3-b]pyrazin-8-yl]-(1,2,2-trimethylpropyl)-amine,
[6-fluoro-4-oxy-7-(2,4,6-trifluorophenyl)-pyrido[2,3-b]pyrazin-8-yl]-(3,3,3-trifluoro-1-methylpropyl)-amine,
[7-(2,4-dichloro-phenyl)-6-fluoro-4-oxy-pyrido[2,3-b]pyrazin-8-yl]-(1,2-dimethylpropyl)-amine, [7-(2,4-dichloro-phenyl)-6-fluoro-4-oxy-pyrido[2,3-b]pyrazin-8-yl]-(1,2,2-trimethyl-propyl)-amine,
[6-chloro-4-oxy-7-(2,4,6-trifluorophenyl)-pyrido[2,3-b]pyrazin-8-yl]-(1,2-dimethylpropyl)-amine, and
[6-chloro-4-oxy-7-(2,4,6-trifluorophenyl)-pyrido[2,3-b]pyrazin-8-yl]-(1,2,2-trimethyl-propyl)-amine.

10. A process for the preparation of a compound of formula II wherein R, R¹, R², R⁷ and R⁸ are as defined for compound of formula I in claims 1 to 8, 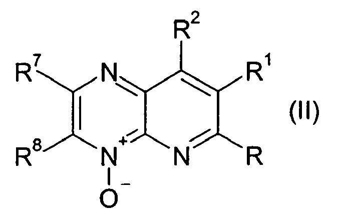 which comprises reacting a compound of formula V wherein R, R¹, R², R⁷ and R⁸ are as defined for compound of formula I in claims 1 to 8 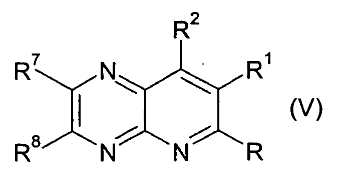 with a percarboxylic acid.

11. A process for the preparation of a compound of formula IV wherein R, R¹, R², R⁷ and R⁸ are as defined for compound of formula I in claims 1 to 8, 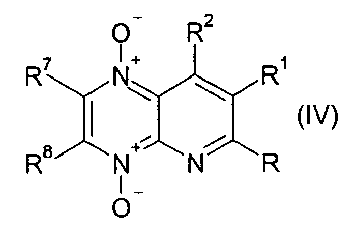 which comprises reacting a compound of formula II wherein R, R¹, R², R⁷ and R⁸ are as defined for compound of formula I in claims 1 to 8 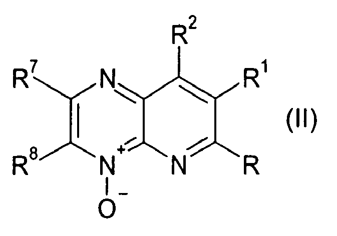 with a percarboxylic acid.

12. A process for the preparation of a compound of formula III wherein R, R¹, R², R⁷ and R⁸ are as defined for compound of formula I in claims 1 to 8, 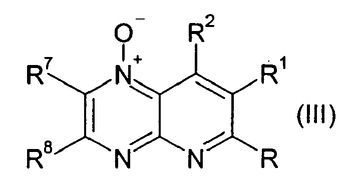 which comprises reacting a compound of formula IV wherein R, R¹, R², R⁷ and R⁸ are as defined for compound of formula I in claims 1 to 8 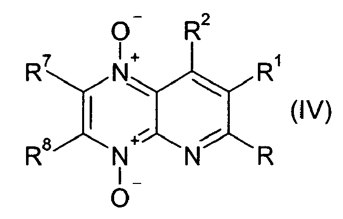 with a reducing agent.

13. A fungicidal composition for controlling or protecting against phytopathogenic microorganisms, comprising as active ingredient at least one compound as defined in any one of claims 1 to 9, in free form or in agrochemically usable salt form, and at least one adjuvant.

14. A composition according to claim 13, which comprises at least one additional fungicidally active compound, preferably selected from the group consisting of azoles, pyrimidinyl carbinoles, 2-amino-pyrimidines, morpholines, anilinopyrimidines, pyrroles, phenylamides, benzimidazoles, dicarboximides, carboxamides, strobilurines, dithiocarbamates, N-halomethylthiotetrahydrophthalimides, copper-compounds, nitrophenols, organo-phosphor-derivatives or pyridazine-derivatives.

15. The use of a compound of formula I as defined in claim 1 for controlling or preventing infestation of plants, seeds or non-living materials by phytopathogenic microorganisms.

16. A method of controlling or preventing an infestation of crop plants, harvested food crops or non-living materials by phytopathogenic or spoilage microorganisms or organisms potentially harmful to man, which comprises the application of a compound as defined any one of claims 1 to 9, as active ingredient to the plant, to parts of the plants or to the locus thereof, to seeds or to any part of the non-living materials.

17. A method according to claim 16, wherein the phytopathogenic microorganisms are fungal organisms.
